(19) Europäisches Patentamt
European Patent Office
Office européen des brevets

(11) **EP 2 314 261 A1**

(12) **EUROPEAN PATENT APPLICATION**

(43) Date of publication:
**27.04.2011 Bulletin 2011/17**

(51) Int Cl.:
*A61F 13/15* (2006.01)

(21) Application number: **09013198.8**

(22) Date of filing: **20.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **The Procter & Gamble Company Cincinnati, OH 45202 (US)**

(72) Inventors:
• **Beruda, Holger**
  **65824 Schwalbach am Taunus (DE)**
• **Dziezok, Peter**
  **65239 Hochheim (DE)**

• **Ehrnsperger, Bruno Johannes**
  **65812 Bad Soden (DE)**
• **Engel, Roland**
  **65843 Sulzberg / TS (DE)**
• **Schmidt, Matthias**
  **65510 Idstein (DE)**
• **Stelzig, Lutz**
  **60489 Frankfurt/Main (DE)**
• **Thomann, Maike**
  **65830 Kriftel (DE)**

(74) Representative: **Heide, Ute et al**
  **Procter & Gamble European Service GmbH**
  **Sulzbacher Strasse 40-50**
  **65824 Schwalbach am Taunus (DE)**

(54) **Method for improving the capacity profile of an absorbent core**

(57) A method for improving the capacity profile of absorbent cores used in absorbent hygiene articles is provided. The method comprises the steps of collecting and analyzing used absorbent articles.

Fig. 1

**Description**

FIELD OF THE INVENTION

The invention generally relates to a method to be used for absorbent articles such as diapers or sanitary napkins. The absorbent articles referred to comprise absorbent cores. More specifically, the invention relates to a method for improving the capacity profile of an absorbent core.

BACKGROUND OF THE INVENTION

Absorbent articles commonly comprise absorbent cores for storing fluids discharged from the body. Absorbent cores typically comprise an absorbent material, which may be equally distributed throughout the absorbent core (herein also referred to as "flat profile").

However, for example depending on anatomy and the resulting point of liquid insult, or due to typical movement of a wearer, a flat profile may not be ideal. Some areas of an absorbent core will more likely be exposed to bodily fluids than others. Therefore, there may be areas where a higher amount of absorbent material is desirable and other areas where a comparably lower amount of absorbent material may be sufficient.

Accordingly, there is a general need to improve the distribution of absorbent material in absorbent cores.

Also, to reduce production cost and for environmental reasons, it may be desirable to reduce the overall amount of absorbent material used in absorbent cores.

Furthermore, a disadvantageous distribution of absorbent material throughout the core may increase leakage of the absorbent article.

Leakage of absorbent articles such as diapers or sanitary napkins is a common and often addressed problem in the art of developing such articles.

Leakage may be expressed as the percentage of absorbent articles that show leakage of bodily fluids during a wearing period. The actual leakage percentage in use is typically determined by conducting so-called "diary tests" wherein consumers test absorbent articles and are asked to document for each of the tested articles if it leaked or not. In more specific tests, the panelists may also be asked to specify where on the article leakage has been observed.

Even though such tests give a general impression of the performance of absorbent articles, they rarely provide sufficient information for a person skilled in the art to identify the cause of leakage.

Another drawback of diary testing is that the collected information is not entirely objective, but may be superimposed by subjective emotions and impressions of the test person or care giver using it. To give an example, an incontinent adult may perceive leakage occurring in public stronger compared to leakage occurring in a not embarrassing and easy to handle situation at home.

It is therefore not straight-forward to extract more information from diary testing than whether the absorbent article leaked, or not.

Moreover, diary testing has typically to be repeated with prototypes of every possibly improved option of absorbent articles. This requires the production of a significant number of prototypes which is technically challenging and expensive.

Another method for collecting information about the performance of absorbent articles is called "mannequin testing". In stich a test absorbent articles are applied to mannequins and gushes of bodily fluids are simulated with test liquids and observed. In some instances, such mannequins can even be programmed to make certain typical movements.

In any case, mannequin testing typically yields data for idealized wearing conditions and thus does not fully reflect real in-use conditions. Also, mannequin testing usually requires a lot of time and equipment.

In sum, there is a general need for a method providing more detailed and reliable information about the leakage performance of absorbent articles. Furthermore, there is a need to obtain such information in a fast and comparably inexpensive way. There is also a need for the information to be linkable to features of an absorbent article which, it a subsequent step, can be adapted accordingly.

Furthermore, there is a need for a method to identify optimized options of absorbent articles to limit the number of prototypes to be tested.

Additionally, there is a need for a method allowing the input of specific boundary conditions, such as process constraints, to identify an improved absorbent article meeting these conditions.

SUMMARY OF THE INVENTION

A method for improving the capacity profile of absorbent cores in absorbent articles is provided. The absorbent cores have a first capacity profile, a x-axis defining a x-direction along their length and a y-axis defining a y-direction along their width.

The method comprises the following steps:

A) collecting the absorbent articles after use
B) analyzing the cores of the collected absorbent articles and determining a load profile per absorbent core by determining the amount of absorbed liquid in x-direction and in y-direction, or only in x-direction
C) calculating an improved second capacity profile based on the load profiles of step B).

The method described herein may be used to improve the capacity profile of absorbent cores with respect to any aspect selected from a group consisting of overall capacity, capacity profile, overall amount of absorbent material, distribution of absorbent material, leakage probability and combinations thereof.

BRIEF DESCRIPTION OF THE DRAWING

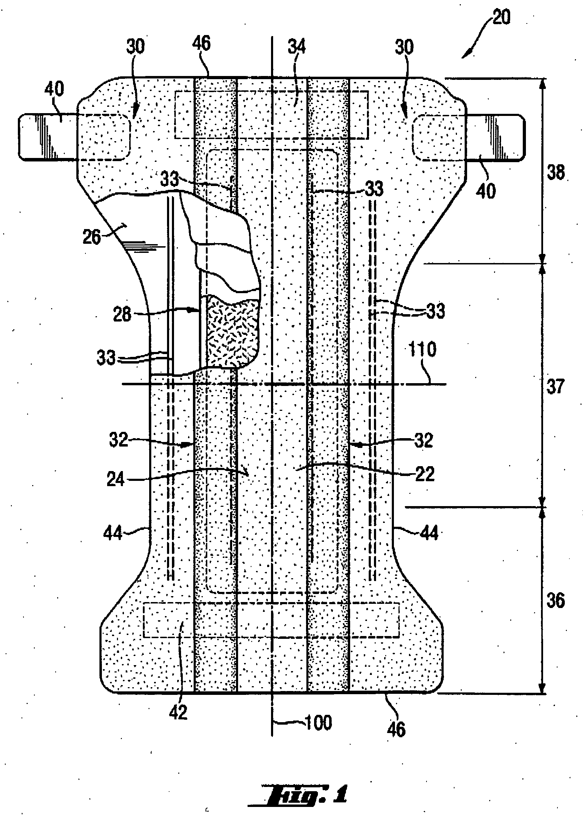

Figure 1 shows a diaper as an exemplary embodiment of an absorbent article.

DETAILED DESCRIPTION OF THE INVENTION

Definition of terms

*"Absorbent article* herein refers to an article comprising an absorbent core. Generally, absorbent articles are capable of absorbing and storing exudates discharged from the body.

The term absorbent article generally refers to an article placed against or in proximity to the body of a wearer to absorb and contain the exudates discharged from the body. Typical absorbent articles may be diapers, training pants, sanitary napkins, panty liners, adult incontinence briefs, adult incontinence undergarments, absorbent inserts and the like.

Typically, the absorbent articles referred to herein are disposable, for example disposable diapers.

"*Diaper*" refers to an absorbent article that is intended to be worn by wearer about the lower torso to absorb and contain exudates discharged from the body.

Diapers are typically worn by infants (e.g. babies or toddlers) and may be provided with fastening elements or in pant form having fixed sides and leg openings. Pant-like diapers are placed in position on the wearer by inserting the wearer's legs into the leg openings and sliding the pant-like diaper into position about the wearer's lower torso.

*"Disposable"* refers to items that are intended to be discarded after a limited number of uses, frequently a single use (i.e., the original absorbent article as a whole is not intended to be laundered or reused as an absorbent article, although certain materials or portions of the absorbent article may be recycled, reused, or composted). For example, certain disposable absorbent articles may be temporarily restored to substantially full functionality through the use of removable/replaceable components but the article is nevertheless considered to be disposable because the entire article is intended to be discarded after a limited number of uses.

*"Absorbent core"* refers to a member of an absorbent article that is intended to absorb and store exudates discharged from the body.

The absorbent core comprises absorbent material and, optionally, a core wrap. Optionally, the absorbent core may comprise a core glue, such as a micro-fiber glue.

*"Absorbent material* " refers to liquid absorbent materials such as for example soft materials providing a rather fluffy structure with a lot of empty space, such as comminuted wood pulp, creped cellulose wadding, chemically stiffened, modified ore cross-linked cellulosic fibers all of which are herein generally referred to as *"airfelt".* Absorbent material also refers to superabsorbent polymer material (SAP), such as super absorbent polymer particles, fibers or foams and mixtures of superabsorbent polymer material with airfelt.

In certain embodiments, absorbent materials may refer to paper towels, tissue, nonwovens, absorbent foams/ sponges, cloth and the like.

The absorbent material may advantageously be compressible, conformable, non-irritating to the wearer's skin, and capable of absorbing and retaining liquids such as urine and certain other body exudates.

*"Core wrap"* refers to fabrics used to enclose materials and components comprised by the absorbent core, such as absorbent material and, if present, materials optionally comprised by the core such as core glue. Frequently, the core wrap, is a nonwoven fabric comprising synthetic fibers. In some embodiments, the core wrap itself may be made of an absorbent material such as tissue paper or the like. Therefore, in certain embodiments, the core wrap as well as the absorbent material may contribute to the overall capacity of the absorbent core.

*"Nonwoven fabric"* refers to a manufactured web of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin. They may be staple or continuous filaments or be formed in situ. The terms "nonwoven fabric" and "nonwoven web" are used interchangeably. The basis weight of nonwoven fabrics is usually expressed in grams per square meter ($g/m^2$) and can be determined according to EDANA method 40.3-90. Generally, nonwoven fabrics may comprise fibers made by nature (natural fibers), made by man (synthetic fibers), or combinations thereof. Example natural fibers include but are not limited to: animal fibers such as wool, silk fur, and hair; vegetable fibers such as cellulose, cotton, flax, linen, and hemp; and certain naturally occurring mineral fibers.

*"Core glue"* herein refers to glue used in the absorbent core to immobilize the absorbent material. Typically, the core glue is applied in microfibers and enlaces the absorbent material. Core glues may be thermoplastic hot melt adhesives. The term hot melt adhesive refers to an adhesive applied from the melt and gaining strength upon solidification (see "Adhesion and Adhesives Technology: An Introduction" by Alphonsus V. Pocius (Hanser publishers Munich, 1997).

*"Capacity"* herein refers to the maximum amount of liquid that can be absorbed by a given amount of material, a component or a part of a component, such as the absorbent material, an absorbent core or a zone of an absorbent core. The capacity is given in g. The specific capacity of a material refers to its capacity divided by its weight and is thus given in g/g.

*"Capacity profile"* herein refers to a map of the capacity in different locations of an absorbent core. In certain embodiments the capacity profile is a 2-dimensional distribution profile wherein the capacity in z-direction has been integrated and thus the distribution of absorbent material in the x,y-plane is given. In other embodiments, the amount of absorbent is integrated in the z-direction and in the x-direction and thus the distribution of absorbent material along the y-axis is given.

*"First capacity profile"* refers to the capacity profile of an absorbent core which is intended to be improved by the method of the present invention.

*"Second capacity profile"* refers to the improved capacity profile derived by the method of the present invention.

*"Load"* refers to the amount of liquid (in g) which has been absorbed to the whole absorbent core, or a part of the absorbent core such as a zone, during its use.

*"Saturation"* refers to the quotient of load in g divided by capacity in g (dimensionless). Saturation can be given for the whole absorbent core or for a part of the absorbent core, such as a zone. When saturation is given for a part of the absorbent core, the values of load and capacity shall be taken from corresponding parts of a used absorbent core (load) and an un-used absorbent core (capacity) respectively.

*"Corresponding part"* refers to parts or zones of different absorbent cores having the same size and location in the x,y-plane of the respective absorbent cores.

*"Used absorbent article"* herein refers to absorbent articles which have actually been used by a wearer. For example a used diaper has been worn by a baby and the baby urinated at least once into the diaper.

*"Leakage probability"* denotes the fraction of articles that leaked. Per definition the leakage probability equals 1 in case all articles leaked and 0 in case none of the absorbent articles leaked.

*"Zones of absorbent cores"* refers to virtual zones an absorbent core has been divided into to facilitate calculations. In such embodiments, each absorbent core is virtually divided into a certain number of zones having a certain length in x-direction. Optionally, the zones have identical lengths in x-direction. In some embodiments, the absorbent cores may be virtually divided into any number of zones between 3-12, such as 8.

*"Comprise, " "comprising "* and *"comprises"* is an open ended term that specifies the presence of what follows e.g. a component but does not preclude the presence of other features, elements, steps or components known in the art, or disclosed herein.

## Exemplary absorbent articles

Figure 1 is a plan view of a diaper 20 as an embodiment of an absorbent article. The diaper is shown in its flat out, uncontracted state (i.e., without elastic induced contraction). Portions of the structure are cut away to more clearly show the underlying structure of the diaper 20. The portion of the diaper 20 that contacts a wearer is facing the viewer.

The diaper 20 has a length along a longitudinal axis 100 and a width along a transverse axis 110. The periphery of the diaper 20 is defined by the outer edges of the diaper 20 in which the longitudinal edges 44 run generally parallel to the longitudinal axis 100 of the diaper 20 and the end edges 46 run generally parallel to the transverse axis 110 of the diaper 20.

The chassis 22 of the diaper 20 in Figure 1 comprises the main body of the diaper 20. The chassis 22 comprises an outer covering including a liquid pervious topsheet 24 and/or a liquid impervious backsheet 26. The entire absorbent core 28 is encased between the topsheet 24 and the backsheet 26.

Optionally, the diaper 20 comprises an acquisition system to acquire and temporarily store fluids discharged from the body. Such an acquisition system may be deposited between the absorbent core 28 and the topsheet 24.

The chassis 22 may further include side panels 30, leg cuffs 32 with elastic members 33 and a waist feature 34. The leg cuffs 32 and the waist feature 34'typically comprise elastic members.

One end portion of the diaper is configured as the front waist region 36 of the diaper 20. The opposite end portion is configured as the rear waist region 38 of the diaper 20. The intermediate portion of the diaper is configured as the crotch region 37, which extends longitudinally between the front and rear waist regions. The crotch region 37 is that portion of the diaper 20 which when the diaper is worn, is generally positioned between the wearer's legs.

The waist regions 36 and 38 may include a fastening system comprising fastening members 40 preferably attached to the rear waist region 38 and a landing zone 42 attached to the front waist region 36. Alternatively, the rear waist region may be permanently bonded to the front waist region to from a pant-type diaper having a waist opening and two leg openings.

In one embodiment the topsheet of the absorbent garment can be apertured, i.e. the topsheet has a plurality of apertures having an aperture size of at least about 0.2 mm$^2$. The topsheet may have an open area of at least about 10%, the open area being the sum of all apertures. The method to determine the aperture size and open area of the apertured topsheet is disclosed in EP 0953324,

The diaper may also include other features as are known in the art including front and rear ear panels, waist cap features, elastics and the like to provide better fit, containment and aesthetic characteristics.

**Absorbent core**

An absorbent core has two major sides, a first side facing the body af the wearer when the absorbent article is worn and a second side facing the garment when the absorbent article is worn. Accordingly, the first and second side may also be referred to as body-facing and garment facing side.

The absorbent core has a length along a x-axis, a width, smaller than the length, along a y-axis and a height, smaller than the width, along a z-axis. In the cores flat out, uncontracted state the axes x, y and z define a Cartesian coordinate system.

In an absorbent article, the core is generally positioned such that its x-axis is substantially parallel to the length (longitudinal axis) of the absorbent article and its y-axis is substantially parallel to the width (transverse axis) of the absorbent article.

The absorbent core has a perimeter line defining its shape in the x,y plane. The perimeter line is substantially symmetrical with respect to a longitudinal centerline running parallel to the x-axis. In other words the longitudinal centerline divides the absorbent core into two substantially mirror symmetrical halves.

In certain embodiments, the absorbent core may be of rectangular shape in the x,y plane. In other embodiments the core may have curved edges. For example, the core may be of an hourglass shape in the x,y plane.

The absorbent core has a first and a second end point defined by the intersecting points of longitudinal centerline and perimeter line. When worn, the absorbent core is intended to be positioned such that the first end point faces towards the front of the wearer and the second end point faces towards the back of the wearer.

The absorbent core may comprise a core wrap and absorbent material. Optionally, the absorbent core comprises one ore more glues, surfactants, binders, colors, pigments, perfume, lotion(s), opacity enhances, nonwovens, odor control materials or materials to increase the dry/wet integrity of the core, such as structural elements.

The core wrap is used to cover the absorbent material. In certain embodiments the absorbent material and, if present, the core glue may either be sandwiched between two separately provided sheets of core wrap material, or may be wrapped by folding one sheet of core wrap material, for example in a C-fold, to envelope the absorbent material and, optionally, the core glue.

In one embodiment the absorbent core may for example comprise as a core wrap a nonwoven fabric. The absorbent material, such as the superabsorbent polymer material may then be deposited on the nonwoven fabric. If present, the core glue may be deposited such that it at least partly covers or enlaces the absorbent material on the nonwoven fabric,

The amounts of materials used in the absorbent core herein are given in % by weight relative to the basis weight of the whole absorbent core including the core wrap. The basis weight of the absorbent core is given in g/cm$^2$. The basis weight may be determined by weighing the whole absorbent core. The obtained weight is then divided by the area enclosed by the perimeter line.

The absorbent core may comprise a relatively high amount of superabsorbent polymer material of more than 80%, 85%, 90%, or 95% by weight of the absorbent core.

In certain embodiments, the absorbent core comprises less than 20%, or 15% or 10% or 5% by weight of the absorbent core of airfelt material.

In one embodiment, the absorbent core may be substantially free of, or completely free of airfelt material wherein *"substantially free of"* means that less than 1% by weight of the absorbent core comprises airfelt material and *"completely free of"* means that 0% by weight of the absorbent core consist of airfelt material.

According to certain embodiments, the absorbent core consists essentially of superabsorbent polymer material, core glue and a core wrap. In such an embodiment the amounts of these materials may add up to present up to 99%, or even 100% by weight of the absorbent core.

**Method of improving the capacity profile of an absorbent core**

The method described herein can be used for improving various aspects of an absorbent core, for example:

- the leakage probability of absorbent articles can be reduced

- the distribution of the absorbent material within the absorbent core can be improved to use the absorbent material more effectively.

- the overall amount of absorbent material used in the absorbent core can be reduced.

The method may also be used for improving combinations of these aspects.
Exemplary embodiments are:

1) Reducing or minimizing the overall amount of absorbent material by reducing the overall capacity while keeping the leakage probability constant. In one embodiment the value of the leakage probability of the initial absorbent cores is maintained.

2) Reducing or minimizing the leakage probability while keeping, the overall amount of absorbent material constant by keeping the overall capacity constant. In one embodiment the value of the capacity of the initial absorbent cores is maintained.

The present inventors found that the observed leakage of an absorbent article can be correlated with the time liquid moves freely in the article after a discharge of bodily fluids (also referred to as "time of free liquid). Accordingly, a reduced time of free liquid can lead to reduced leakage of an absorbent article. Further, the inventors found that the time of free liquid can be correlated with the load profiles of used absorbent cores. Also, while for some embodiments of the present method the time of free liquid actually needs to be calculated, there are specific embodiments wherein such a calculation is not needed. However, the present method is based on the assumption that leakage probability and the time of free liquid are correlated to the actual load profiles of absorbent cores after use and therefore requires determining these load profiles and calculating therefrom an improved second capacity profile.

Determining the first capacity profile

The first capacity profile of an absorbent core may be determined using the procedure described below (see step B) for determining the load profile. The procedure for determining the first capacity profile differs from the procedure for determining the load profile in that absorbent cores which have been saturated with deionized water comprising 0.9% (by weight) NaCl at room temperature 22°C±2°C are used instead of the "used absorbent cores". Saturating the cores is done by fully immersing the cores into an large excess amount of deionized water comprising 0.9% (by weight) NaCl at room temperature 22°C±2°C for 30 minutes, then carefully (without destroying the core) putting the core onto a horizontal grid for 10 minutes at room temperature and 40-60% relative humidity.

**Step A)** Collecting the Absorbent articles after having been used

When collecting used absorbent articles for analysis it should be sought to collect enough samples to get results representative for the entire range of usage conditions. For example, at least 250, or at least 400 absorbent cores should be analyzed. In certain embodiments up to 2000 absorbent cores may be analyzed.
The used absorbent cores may be selected such that they reflect the group of wearers and the usage conditions for which the improved articles are intended.
For example, a group of wearers may be selected according to age, gender, body height, body weight, body shape, sleeping habits, geographical region and combinations thereof.
For example, to reflect usage conditions, the absorbent cores may be selected according to the time the absorbent articles have been worn, their weight after use, whether they contain bowel movement or not, the time of day they have been worn, such as during the day or over night.
For example, to improve an absorbent core for an overnight diaper, the collected used absorbent articles should have been worn over night. Further, the panelists may be selected according to their sleeping habits, e.g. 50% sleeping mostly on the stomach and 50% sleeping mostly on the back.

In certain embodiments the collected absorbent articles are identical, wherein *identical* refers to articles of the same *"product type"* (different product types are for example taped diapers vs. pant type diapers) having the same features such as identically constructed absorbent cores, same size, same form and are assembled from the same additional components. As used herein, the term "same features" is meant to exclude pure design or esthetic elements.

Herein, *"additional components"* of a diaper include commonly used components such as the topsheet, backsheet, fastening system, elastics, acquisition layers and cuffs, but not the absorbent core.

In other embodiments the absorbent articles are similar, wherein *"similar absorbent article"* refers to different options of absorbent articles of the same product type but may differ in one or more features selected from the group consisting of size, form and additional components.

In certain embodiments, the similar absorbent articles may only differ in one of these features. In one embodiment the similar articles only differ in their size.

In embodiments wherein the absorbent articles are similar, enough samples of each of the similar, options of the articles should be collected and analyzed. In certain embodiments, the same number of absorbent cores with respect to each option of the similar articles may be analyzed. For example, at least 100, or at least 200, or even at least 400 absorbent articles of each of the similar options may be analyzed.

Preferably, the collected absorbent articles should not be stored over an extended period of time before being analyzed. In case the articles are stored, they should be stored separately in sealed plastic bags to avoid evaporation. In such cases, the sealed articles should be stored at $22\pm2°$C.

**Step B)** Analyzing absorbent cores of collected absorbent articles

Prior to analysis, the absorbent cores are carefully separated from the rest of the used absorbent article and put in a flat configuration.

By carefully it is meant that the absorbent cores shall not be folded, twisted, pulled or squeezed but shall be removed in a non-disruptive way. In other words, the cores should not be treated in a way causing ruptures, deformation and the like. Deformed or ruptured cores shall not be used for the method described herein.

The load profile of an absorbent core may be determined by any technique suitable for determining the amount of absorbed liquid in x-direction (1-dimensional) or x,y (2-dimensional), or x,y,z (3-dimensional) direction of the absorbent core.

Exemplary suitable techniques are gravimetric analysis or x-ray imaging.

The load profile shall be suitable for determining gush peaks, gush points and their corresponding gush volumes.

*"Crush peak"* herein refers to a peak in the load profile having a maximum or a local maximum. The locations where the load profiles have a maximum, or local maxima are referred to as *"gush points* ". The *"gush volume"* $v_g$ of a gush peak is calculated from the load profile as follows:

a) If an absorbent core shows only one gush peak the gush volume is equal to' the overall load of the absorbent core.

b) If an absorbent core shows more than one gush peak, overall load of the absorbent core equals the sum of all gush volumes detected in that absorbent core. In such cases, the ratio between the peak heights (the value of the load at the maximum) is equal to the ratio of the gush volumes. E.g.for three gushes with peak heights $p_1$, $p_2$, $p_3$ and an overall load of V; the gush volumes $v_1$, $v_2$, $v_3$ ; are calculated as:

$$V = v_1 + v_2 + v_3$$

$$v_1 : v_2 : v_3 = p_1 : p_2 : p_3$$

As an exemplary embodiment the gravimetric analysis of a single absorbent core yielding a 2-dimensional load profile will now be described.

A used absorbent core is placed on a flat surface and cut into squares of identical size, such as 1x1cm squares parallel to the x-and y-axis. Each core square is marked corresponding to its location in the x,y plane, weighed using a balance with an accuracy of at least $\pm$ 0.05 g and the weight is recorded. The amount of absorbed liquid (load of the core square) for said location is then determined by subtracting the weight of a corresponding un-used core square.

It should be borne in mind that typically even very small babies do not urinate more frequent than about five to eight times into one diaper. Therefore, detecting an artificially high number of local maxima may indicate that the data contain noise. In such a case it may be advantageous to apply smoothening routines in order to reduce noise prior to determining gush peaks, gush points and gush volumes from the load profiles.

For example, in case the load values are determined by a gravimetric method, for each core the loads per core square may be recorded corresponding to their location in the xy-plane in a matrix.

In case of a one dimensional profile of the core is desired, the values are added along the y-direction.

Estimation of time of free liquid and leakage probability

Without wishing to be bound by theory, it is believed that one factor the leakage probability of an absorbent article depends on is the amount of time liquid discharged from the body moves freely in the article (i.e. before being absorbed and stored by the absorbent core). This time is referred to herein as *"time of free liquid" ($t_{free}$)*. Methods for estimating the time of free liquid are given in the below.

The leakage probability may depend on the number of gush peaks per absorbent core, their gush volume $v_g$ and their location in the load profile (gush point).

For the method described herein, the time of free liquid may be estimated and recorded for every gush peak in each load profile separately.

One way of estimating the time of free liquid and the leakage probability with data obtainable from the load profile will now be described.

The calculation will be described for absorbent cores which have been virtually divided into k zones of equal length in x-direction.

Without wishing to be bound be theory, it is believed that the time of free liquid for a zone k wherein a gush peak has been detected is proportional to the volume of the gush (gush volume $v_g$) and the saturation $S(k)$ in this zone to a power of n. Accordingly, for this embodiment, the time of free liquid can be estimated as follows:

$$t_{free}\left(v_g, l(k), Cap(k)\right) = c \cdot v_g \cdot S(k)^n = c \cdot v_g \cdot \left(\frac{l(k)}{Cap(k)}\right)^n \qquad (1)$$

When calculating the time of free liquid according to (1) only the capacity in the zone were the gush occurred contributes to the time of free liquid.

For $n_j$ analyzed absorbent cores (j being the index referring to the core) and $n_i(j)$ detected gush points per analyzed core j (i being the index referring to the gush points), the leakage probability depending on the time of free liquid according to equation (1) may be estimated by the formulas (2) and (3) below:

$$P_{leak}\left(t_{free}\left(v_g, l(k), Cap(k)\right)\right) = \frac{1}{n_j} \cdot \sum_{j=1}^{n_j} \sum_{i=1}^{n_i(j)} \frac{1}{1 + \exp\left(a - \frac{b}{c} t_{free}\left(v_g, l(k), Cap(k)\right)\right)} \qquad (2)$$

$$P_{leak}\left(v_g, l(k), Cap(k)\right) = \frac{1}{n_j} \cdot \sum_{j=1}^{n_j} \sum_{i=1}^{n_i(j)} \frac{1}{1 + \exp\left(a - b \cdot v_g(i,j) \cdot \left(\frac{l_j(k_{i,j})}{Cap(k_{i,j})}\right)^n\right)} \qquad (3)$$

In another embodiment (1a) the time of free liquid may be estimated taking into account the capacity in the zones next to the zone of the gush peak *("neighboring zones")* according to equation (1a). In (1a) not only the capacities of the zone were the gush occurs, but also the capacities of the neighboring zones, weighted by their distance to the gush peak contribute to the time of free liquid.

$$t_{free}(i,j) = c \cdot V_G \cdot \left\{ \left( \frac{l(k_{ij})}{Cap(k_{ij})} \right)^2 + w \cdot \left[ f_1(i,j) \cdot \left( \frac{l(Max(1,k_{ij}-1))}{Cap(Max(1,k_{ij}-1))} \right)^2 + f_2(i,j) \cdot \left( \frac{l(Min(k_{max},k_{ij}+1))}{Cap(Min(k_{max},k_{ij}+1))} \right)^2 \right] \right\}$$

(1a)

The symbol "Min(a,b)" in equation (1a) refers to the minimum between the two values in bracket (a,b), the symbol Max(a,b) refers to the maximum of the two values in bracket (a,b). For zones of equal length $l_{zone}$, functions $f_1$ and $f_2$ in (1a) are given by;

$$f_1(i,j) = 1 - \frac{p_{ij}}{l_{zone}} + INT\left( \frac{p_{ij}}{l_{zone}} \right)$$

$$f_2(i,j) = \frac{p_{ij}}{l_{zone}} - INT\left( \frac{p_{ij}}{l_{zone}} \right)$$

(1b)

$P_{ij}$ denotes the position (along the x-axis) of the $i^{th}$ gush peak in the $j^{th}$ core. The symbol INT(x) denotes the integer value of a real number x with everything behind the decimal point being subtracted from the number. E.g. INT (3.89) =3, or INT(3.14)=3.

l(k) refers to the load in zone k, Cap(k) refers to the core capacity in zone k. Zone $k_{ij}$ is the zone were the $i^{th}$ gush from absorbent core j occurred. $p_{ij}$ is the peak position of the $i^{th}$ gush in absorbent core j.

In another embodiment, it is assumed that the leakage probability depends on the time of free liquid in a S-shaped type of curve. In this embodiment, the leakage probability can be estimated according to equation (2a):

$$P_{leak}(t_{free}) = \frac{\dfrac{1}{1+\exp(a - b \cdot t_{free})} - \dfrac{1}{1+\exp(a)}}{1 - \dfrac{1}{1+\exp(a)}}$$

(2a)

Accordingly, for j analyzed absorbent cores and i detected gush points, the leakage probability depending on the time of free liquid and the capacity may be estimated by plugging (1) or (1a) into (2a) giving the formulas (3a) and (3b) respectively:

$$P_{leak}\left(t_{free}(v_g, l(k), Cap(k))\right) = \frac{1}{n_j} \cdot \sum_{j=1}^{n_j} \sum_{i=1}^{n_i(j)} \frac{\dfrac{1}{1+\exp\left(a - b \cdot v_g(i,j) \cdot \left( \dfrac{l_j(k_{i,j})}{Cap(k_{i,j})} \right)^n \right)} - \dfrac{1}{1+\exp(a)}}{1 - \dfrac{1}{1+\exp(a)}}$$

(3a)

$$P_{test}\left(t_{free}\left(v_g, l(k), Cap(k)\right)\right) = \frac{1}{n_j} \cdot \sum_{j=1}^{n_j} \sum_{i=1}^{n_i(j)} \frac{\frac{1}{1+\exp\left[a - b \cdot V_G \cdot \left[\left(\frac{l(k_{ij})}{Cap(k_{ij})}\right)^2 + w \cdot \left[f_1(i,j) \cdot \left(\frac{l(Max(1,k_{ij}-1))}{Cap(Max(1,k_{ij}-1))}\right)^2 + f_2(i,j) \cdot \left(\frac{l(Min(k_{max},k_{ij}+1))}{Cap(Min(k_{max},k_{ij}+1))}\right)^2\right]\right]\right]} - \frac{1}{1+\exp(a)}}{1 - \frac{1}{1+\exp(a)}}$$

$$(3b)$$

Where in equations (2), (3), (3a) and. (3b):

- Index j refers to the jth absorbent core (e.g. for 400 used absorbent cores j =1,..., 400),

- Index i refers to the ith gush peak in a given absorbent core j (typically i= 1,..,3),

- Index k refers to the kth zone (in x-direction) of an absorbent core j (e.g. each of the absorbent cores may be virtually divided in 8 zones, k =1,...., 8 of equal length).

- $n_j$ denotes the total number of analyzed used absorbent cores (e.g., $n_j$ = 400 for 400 used absorbent cores).

- $n_i(j)$ denotes the number of gush peaks in used absorbent core j (e.g. $n_i$ (j=12) = 3) means that the 12th used absorbent core had 3 gushes.

- $v_G(i,j)$ denotes the gush volume of gush peak i in absorbent core j.

- $k_{ij}$ is the zone were gush number i in absorbent core j occurred

- $lj(k_{ij})$ is the load in zone $k_{ij}$ of absorbent core j.

- Cap(k) is the capacity in zone k.

With the empirical constants:

- a = 3.42

- b=1/(200g)

- c=0.5g/s

- n=2

While the already described embodiments for estimating the time of free liquid are generally preferred, another option (2b) for estimating the time of free liquid, which is especially useful in case the first capacity profile is a reverse profile (i.e. the capacity at the ends of the absorbent core is higher compared to the center of the absorbent core), will now be explained:

$$t_{free}(i,j) = \tilde{c} \cdot \left(\frac{V_G(i,j)}{w \cdot f_1(i,j) \cdot Cap(k_{ij}-1) + Cap(k_{ij}) + w \cdot f_2(i,j) \cdot Cap(k_{ij}+1)}\right)^n \qquad (2b)$$

Useful values in equation (2b) are:

$\bar{c}$ =100$s$

w=1

n=2

$$f_1(i,j) = 1 - \frac{p_{ij}}{5cm} + INT\left(\frac{p_{ij}}{5cm}\right)$$

$$f_2(i,j) = \frac{p_{ij}}{5cm} - INT\left(\frac{p_{ij}}{5cm}\right)$$

When the time of free liquid is estimated according to equation 2b, the leakage probability can be estimated by:

$$P_{leak} = \frac{1}{n_j} \cdot \frac{\displaystyle\sum_{j=1}^{n_z} \sum_{i=1}^{n_z(j)} \frac{1}{1+\exp\left(a - \tilde{b} \cdot t_{free}(i,j)\right)} - \frac{1}{1+\exp(a)}}{1 - \frac{1}{1+\exp(a)}} \qquad (4)$$

With

a = 3.42 and $\tilde{b}$ = 0.01/s.

Even though several methods of estimating the time of free liquid have been described, it should be noted that data obtained from a set of used absorbent cores should, be analyzed according to one of the described methods (i.e. the methods shall not be used simultaneously).

Step C) Calculating an improved second capacity profile

Several embodiments of improving the capacity profile of an absorbent core will now be described.
In certain embodiments, the second capacity profile may be calculated from the first capacity profile by minimizing the leakage probability.
In other embodiments, the second capacity profile may be calculated by minimizing the capacity ,

1) Minimizing $P_{leak}$

The leakage probability may be minimized using certain boundary conditions.
Exemplary boundary conditions may be an upper limit of the overall capacity, or a combination of an upper and lower limit of the overall capacity, or a constant target value of the overall capacity.
In embodiments where the absorbent core has been divided into virtual zones, the leakage probability can be minimized by varying the capacity of the zones under given boundary conditions.
In some embodiments boundary conditions may be set for each zone individually. For example, different upper and / or lower limits of capacity may be set for each zone individually, or the capacity for some, excluding at least two, zones may be set to a constant value.

ii) Minimizing capacity

The capacity may be minimized by setting an upper limit for the leakage probability.
In one embodiment where the absorbent core has been divided into virtual zones, the second capacity profile may be calculated from the first capacity profile by setting an
upper limit for the leakage probability and minimizing the overall capacity while varying the capacity in the individual zones.
In some embodiments, the leakage probability may be set to an upper limit and the capacity in certain zones may be set to a maximum and / or minimum value. The capacity in all zones will then be varied until the overall capacity has reached a minimum.

In some embodiments, the leakage probability may be set to an upper limit and the capacity in certain, but excluding at least two, zones may be set to a constant value. The capacity of the remaining zones will then be varied until the overall capacity reaches a minimum.

iii) Minimizing the amount of absorbent material in the core

It will be assumed that the capacity of an absorbent article is proportional to the specific capacity of the absorbent material in the core. Therefore, as long as the same absorbent material is used, reducing the capacity of the absorbent core reduces the amount of absorbent material needed.

iv) Calculating the second capacity profile from the averaged load profile

The inventors found, that calculating the second capacity profile according to the embodiments described above is applicable to flat and profiled (i.e. non-flat) first capacity profiles. However, under the prerequisite that the first capacity profile is flat and the overall absorbent capacity exceeds the average load ($l_{AVE}$) by art least about 100% (for example the overall capacity should exceed at least about 350 g for a maxi size baby diaper), the second capacity profile may also be calculated from the average load profile and its standard deviation.

Each core i may be virtually divided in k zones with k ranging from 1 to $k_{max}$. The average load ($l_{AVE}$) in such a zone k can now be calculated as:

$$l_{AVE}(k) = \frac{1}{N}\sum_{i=1}^{N} l_i(k) \tag{5}$$

The standard deviation of the average load ($l_{STD}$) in a zone k is calculated as:

$$l_{STD}(k) = \sqrt{\frac{\sum_{i=1}^{N}\left(l_{AVE}(k) - l_i(k)\right)^2}{N-1}} \tag{6}$$

The second capacity profile (Cap(k)) can then be calculated by:

$$Cap(k) = \left(l_{AVE}(k) + 2 \cdot l_{STD}(k)\right) \cdot \frac{Cap_{tot}}{\sum_{k'=1}^{k'=k_{max}}\left(l_{AVE}(k') + 2 \cdot l_{STD}(k')\right)} \tag{7}$$

With zones k ranging from 1 to $k_{max}$ and $Cap_{tot}$ being the overall capacity of the core. If one does not want to specify the overall capacity of the core the capacity profile may also be estimated as (7a):

$$Cap(k) = l_{AVE}(k) + 2 \cdot l_{STD}(k) \tag{7a}$$

METHODS

The solve function of Microsoft Excel (Microsoft Excel 2003, SP3, Version 11.0 Build 8302) may be used for the described calculations according to C) i-iii, such as minimizing leakage probability or capacity under the described conditions.

As is well known to the man skilled in the art, under certain conditions (e.g. if the number of returned absorbent cores under investigation is small) the solution that the solve function from Excel finds as the optimum can depend on the initial conditions for the capacity profile. The desired optimum is the one that is minimal (e.g. for leakage, or for the overall capacity) from all possible initial conditions. Under these circumstances Monte Carlo simulation techniques are suitable to find the initial conditions for the solver that give the smallest minimum.

If should be noted that finding the smallest minimum may not be needed for every application of the method described herein. Thus, in certain embodiments it may be desirable to find a sufficiently small minimum instead of the smallest minimum.

Determination of the specific capacity

Without wishing to be bound by theory it is believed that typically the capacity of an absorbent core is proportional to the amount (mass, $m_{ABS}$) of absorbent material in the absorbent core.

The overall capacity ($Cap_{tot}$) of a core may be calculated from the amount (mass) of absorbent material ($m_{abs}$ times the specific capacity (capacity per mass ($C_{mabs}$) of the absorbent material:

$$Cap_{tot} = C_{mabs} \cdot m_{abs}$$

Herein, the specific capacity $C_{mabs}$ refers to the Centrifuge Retention Capacity (CRC) determined according to EDANA method No. 441.1-99.

EDANA method No. 441.1-99 is used in order to determine the capacity of absorbent materials supplied in powdery form, particulate form or in the form of fibers.

In case the specific capacity of the whole absorbent core or a part of the absorbent core, such as a zone, is determined the method will be modified the following way:

point 6.1.: The size of the teabag is to be selected such that it easily fits the material of which the capacity is to be determined throughout the whole procedure.

For example, for a whole absorbent core the size of the teabag may therefore be larger as given in the EDANA method.

Point 7: In case the capacity of the whole absorbent core shall be determined, the whole absorbent core is carefully removed from the rest of the absorbent article.

The absorbent core as a whole is weighed and used for the rest of the procedure.

In case the capacity of a part of the absorbent core, such as a zone, shall be determined, that part is carefully cut out of the absorbent core. The part as a whole is weighed and fused for the rest of the procedure.

Accordingly, when having derived an improved capacity profile an absorbent core having a mass of absorbent material profile that matches this improved second capacity profile is an absorbent core with an improved capacity profile.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A method for improving the capacity profile of cores in absorbent articles, wherein the cores have a first capacity profile, a x-axis defining a x-direction along their length and a y-axis defining a y-direction along their width; the method comprising the steps of

   A) collecting the absorbent articles after use
   B) analyzing the cores of the collected absorbent articles and determining a load profile per absorbent core by determining the amount of absorbed liquid in x-direction and in y-direction, or only in x-direction
   C) calculating an improved second capacity profile from the load profiles of step B).

2. The method of claim 1, wherein step B) further comprises estimating a leakage probability depending on a time of free liquid $P_{leak}(t_{free})$ from the load profiles and wherein step C) further comprises calculating the improved second capacity profile based on the leakage probability $P_{leak}(t_{free})$ estimated according to step B).

3. The method according to claim 2, wherein the time of free liquid is calculated according to $t_{free} = c \cdot v_G \cdot S(k)^n$; with constant $c$=0.5g/s, gush volume $v_G$, $S(k)$ saturation in zone $k$, $n$=2.

4. The method according to claims 2 or 3, wherein the second capacity profile is calculated by maintaining the leakage probability from the first capacity profile while minimizing the overall capacity.

5. The method according to claim 2 or 3, wherein the second capacity profile is calculated by maintaining the overall capacity while minimizing the leakage probability.

6. The method according claim 1, wherein the first capacity profile is a flat profile and the absorbent cores have an overall capacity of at least 350g; wherein step B) further comprises calculating an averaged load profile from the load profiles of the absorbent cores and wherein the second improved capacity profile in step C) is given by

$$Cap(k) = \left( l_{AVE}(k) + 2 \cdot l_{STD}(k) \right) \cdot \frac{Cap_{tot}}{\sum_{k'=1}^{k'=k_{max}} \left( l_{AVE}(k') + 2 \cdot l_{STD}(k') \right)}.$$

7. The method according to any of the preceding claims, wherein the core is virtually divided in zones of a certain length in x-direction.

8. The method according to claim 7, wherein the capacity in certain, but not all, zones of the absorbent core is set to a prescribed value.

9. The method according to any of the preceding claims, wherein the absorbent articles are similar articles, preferably of the same product type, the articles may be of different sizes, but preferably of the same size.

10. The method according to any of the preceding claims, wherein the absorbent articles are identical.

11. The method according to any of the preceding claims wherein said improved second capacity profile is only in x-direction.

12. The method according to any of the preceding claims, wherein the core having the first capacity profile comprises at least 5g superabsorbent polymer material.

13. The method according to any of the preceding claims, wherein more than 50% of the capacity is provided by superabsorbent polymer material.

14. The method of claim 1, wherein the improved second capacity profile has been improved with respect to an aspect selected from a group consisting of overall capacity, capacity profile, overall amount of absorbent material, distribution of absorbent material, leakage probability and combinations thereof.

15. An absorbent core comprising the 2nd capacity profile obtained by the method of any of the preceding claims.

Fig. 1

**Europäisches Patentamt**

**European Patent Office**

**Office européen des brevets**

## PARTIAL EUROPEAN SEARCH REPORT

**Application Number**

which under Rule 63 of the European Patent Convention shall be considered, for the purposes of subsequent proceedings, as the European search report

EP 09 01 3198

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 447 066 A1 (PROCTER & GAMBLE [US]) 18 August 2004 (2004-08-18) * paragraphs [0014] - [0015], [0021] - [0043]; claim 1; figures 1-8 * ----- | 15 | INV. A61F13/15 |

TECHNICAL FIELDS SEARCHED (IPC)

A61F

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 5 March 2010 | Joly, Florence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 09 01 3198

Claim(s) completely searchable:
-

Claim(s) not searched:
1-14

Reason for the limitation of the search (non-patentable invention(s)):

Article 52 (2)(c) EPC - Scheme, rules and method for performing mental acts

Claims 1-14 are considered as a method for performing a mental act .
In fact , claim 1 is :
A method for improving the capacity profile of cores in absorbent articles,
wherein the cores have a first capacity profile, a x-axis defining a x-direction along their length and a y-axis defining a y-direction along their width; the method comprising the steps of
A) collecting the absorbent articles after use
B) analyzing the cores of the collected absorbent articles and determining a load profile per absorbent core by determining the amount of absorbed liquid in x-direction and in y-direction, or only in x-direction
C) calculating an improved second capacity profile from the load profiles of step B).

Claim 1 is considered as a method for performing mental act. Claim 1 comprises step of reasoning . Collecting, analysing and calculating is just step of a reasoning. No product, no process , no technical features are defined in this claim. Moreover in all the description, just mathematical formula and method for performing mental acts are described.


-----

Further limitation of the search

Claim(s) completely searchable:
-

Claim(s) searched incompletely:
15

Reason for the limitation of the search:

3.1) Claim 15 is an absorbent core comprising the second capacity profile obtained by the method of claim 1 . However the second capacity profile is not defined in terms of technical feature in claim 1. Claim 1 is in fact a method performing mental act. Therefore , the absorbent core is not defined by technical features in claim 15. Moreover in the original description pages 9 and 10, just very general statements on absorbent core are described. Therefore it is not clear for the skilled person to know which features are necessary for obtaining the product of claim 15.

Europäisches
Patentamt

European
Patent Office

Office européen
des brevets

**INCOMPLETE SEARCH
SHEET C**

Application Number

EP 09 01 3198

(article 84EPC).
Therefore claim 15 is considered as claiming any commonly known absorbent core of the prior art. Therefore no meaningful search can be possible as the absorbent core of claim 15 is not specifically defined. Rule 63EPC.
 The search has been done on absorbent core comprising a wrap core and superabsorbent(see original description page  10 lines 3-5).

**ANNEX TO THE EUROPEAN SEARCH REPORT
ON EUROPEAN PATENT APPLICATION NO.**

EP 09 01 3198

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2010

| Patent document cited in search report | | Publication date | Patent family member(s) | | | Publication date |
|---|---|---|---|---|---|---|
| EP 1447066 | A1 | 18-08-2004 | AT | 409447 | T | 15-10-2008 |
| | | | AU | 2004212005 | A1 | 26-08-2004 |
| | | | BR | PI0407432 | A | 24-01-2006 |
| | | | CA | 2515143 | A1 | 26-08-2004 |
| | | | CN | 1741781 | A | 01-03-2006 |
| | | | CN | 101036612 | A | 19-09-2007 |
| | | | CN | 101036613 | A | 19-09-2007 |
| | | | CN | 101036614 | A | 19-09-2007 |
| | | | EG | 23766 | A | 08-08-2007 |
| | | | EP | 1813236 | A2 | 01-08-2007 |
| | | | EP | 1813237 | A2 | 01-08-2007 |
| | | | EP | 1808152 | A2 | 18-07-2007 |
| | | | EP | 1982678 | A1 | 22-10-2008 |
| | | | ES | 2314137 | T3 | 16-03-2009 |
| | | | JP | 2006513823 | T | 27-04-2006 |
| | | | JP | 2007244882 | A | 27-09-2007 |
| | | | JP | 2007222646 | A | 06-09-2007 |
| | | | JP | 2007244883 | A | 27-09-2007 |
| | | | KR | 20050100391 | A | 18-10-2005 |
| | | | MX | PA05007929 | A | 22-02-2006 |
| | | | US | 2007156108 | A1 | 05-07-2007 |
| | | | US | 2004162536 | A1 | 19-08-2004 |
| | | | WO | 2004071363 | A1 | 26-08-2004 |
| | | | ZA | 200505365 | A | 26-04-2006 |

For more details about this annex : see Official Journal of the European Patent Office, No. 12/82

**REFERENCES CITED IN THE DESCRIPTION**

**Patent documents cited in the description**

- EP 0953324 A